(19) Europäisches Patentamt
European Patent Office
Office européen des brevets

(11) EP 2 554 252 B1

(12) **EUROPEAN PATENT SPECIFICATION**

(45) Date of publication and mention of the grant of the patent:
**27.02.2019 Bulletin 2019/09**

(21) Application number: **11765518.3**

(22) Date of filing: **28.03.2011**

(51) Int Cl.:
***A61K 35/14*** *(2015.01)* ***A61M 1/36*** *(2006.01)*
***B01J 20/32*** *(2006.01)*

(86) International application number:
**PCT/JP2011/057678**

(87) International publication number:
**WO 2011/125618 (13.10.2011 Gazette 2011/41)**

(54) **SUBSTRATE FOR LIGAND IMMOBILIZATION AND METHOD FOR PRODUCING SAME**

SUBSTRAT ZUR LIGANDENIMMOBILISIERUNG UND VERFAHREN ZU SEINER HERSTELLUNG

SUBSTRAT POUR L'IMMOBILISATION D'UN LIGAND ET PROCÉDÉ POUR LE PRODUIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2010 JP 2010083455**

(43) Date of publication of application:
**06.02.2013 Bulletin 2013/06**

(73) Proprietor: **Asahi Kasei Medical Co., Ltd.**
**Chiyoda-ku**
**Tokyo 101-8101 (JP)**

(72) Inventors:
- **NAGASAWA, Shinichiro**
  **Tokyo 101-8101 (JP)**
- **FUKUSHIMA, Masakazu**
  **Tokyo 101-8101 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2005/026224**
**WO-A2-2007/024393**
**CA-C- 2 112 411**
**GB-A- 1 529 378**
**JP-A- H07 300 513**
**JP-A- 2000 226 550**
**JP-A- 2007 130 194**
**JP-A- 2009 300 239**
**JP-A- 2010 057 745**
**US-A- 3 671 502**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a substrate for ligand support for supporting affinity ligands and a method for producing the same.

### Background Art

For the purpose of removing particular components from blood, adsorbents in which substances having affinity for the particular components, i.e., ligands, are supported on water-insoluble carriers by covalent bond are clinically widely used. These adsorbents are used in a form in which patient blood is temporarily taken out of the body and the blood or plasma separated with a plasma separator is treated by flowing in the adsorbent and then returned to the patient.

Therefore, if ligands run out of the adsorbent, there is the risk of directly entering the patient body and causing various physiological actions. Accordingly, adsorbents in which the amount of ligands eluted is large cannot be clinically used in terms of safety. Specifically, strengthening the bonding between the water-insoluble carrier and the ligands to decrease the amount of ligands eluted is the most important technical challenge for putting these adsorbents into actual use.

Moreover, the blood compatibility of the adsorbent in such a way that the surface adsorption of proteins, cells (white blood cells, platelets, etc.), and other blood components associated with contact with blood is unlikely to occur is also important. Particularly, for selective adsorbents, a substrate in which the nonspecific adsorption of ones other than the components of interest does not occur in the substrate itself is demanded in order to selectively adsorb the particular components.

Conventionally, medical materials such as polyethylene, polypropylene, polyurethane, nylon, and polyvinyl chloride clinically used are merely ones in which industrial resin materials are diverted for medical purposes, and it is not said that blood compatibility is sufficient. Thus, in order to improve the hydrophilicity and hydrophobicity of medical material surface, a method of forming a graft polymerization layer on the surface, in which a radical is generated by delivering radiation, UV rays, or the like to the medical material surface, performing plasma treatment with arc, direct current glow, radiofrequency, microwaves, corona discharge, UV-ozone treatment, or the like, and allowing a radical-polymerizable monomer to act on the radical to obtain the graft polymerization layer, is widely used. For example, graft polymerization using radiation is proposed in Non Patent Literature 1, and a method of graft-polymerizing methyl methacrylate or acrylic acid using an aqueous polyvinyl acetate solution on polypropylene surface or on polyethylene surface is proposed in Non Patent Literature 2.

Meanwhile, poly-2-methacryloyloxyethyl phosphorylcholine (hereinafter, referred to as MPC) having phosphorylcholine, which is a polar group of phospholipid, in the side chain of the polymer chain is widely known as a betaine compound having polymerizability (e.g., Patent Literatures 1 and 2). It is understood that the MPC polymer is excellent in biocompatibility because of having the same phospholipid of amphoteric type as in biomembranes and has the advantages that plasma proteins are not adsorbed on the surface and that the adhesion, activation, or the like of platelets is not induced. However, 2-methacryloyloxyethyl phosphorylcholine constituting the MPC polymer requires complicated operation for its preparation and has the disadvantages that it is difficult to enhance its purity and that formed crystals deliquesce in a short time. Therefore, 2-methacryloyloxyethyl phosphorylcholine is relatively expensive as a synthetic polymer compound due to its inconvenient handling or other influences.

In contrast, N-methacryloyloxyethyl-N,N-dimethylammonium-$\alpha$-N-methylcarboxy betaine (hereinafter, referred to as CMB) having a glycine-type amphoteric group is proposed as a compound that is relatively inexpensive and can be prepared easily at a large scale (e.g., Patent Literatures 3, 4, and 5). There is the report that a copolymer made of CMB and a polymerizable monomer has such excellent biocompatibility that interaction with biogenic substances such as proteins and blood cells is small (Patent Literature 6). However, a substrate for ligand support including on the substrate surface, a copolymer made of CMB and a monomer having a functional group for ligand support has not been known. Patent literature 7 discloses a polymer grafted on the surface of a water-insoluble carrier, the polymer being obtained from monomers comprising betaine groups. The polymer may be obtained by co-polymerizing the monomers comprising betaine groups and further hydrophilic monomers.

Patent literature 8 discloses a separating material obtained by grafting polymer chains on the surface of a water-insoluble carrier, the polymer chains being produced from a mixture of glycidyl methacrylate and functional monomers. The glycidyl methacrylate monomer is used for binding affinity ligands such as proteins, peptides and antibodies.

## Citation List

### Patent Literature

Patent Literature 1: Japanese Patent Application Laid-Open No. 54-63025
Patent Literature 2: Japanese Patent Application Laid-Open No. 2000-279512
Patent Literature 3: Japanese Patent Application Laid-Open No. 9-95474
Patent Literature 4: Japanese Patent Application Laid-Open No. 9-95586
Patent Literature 5: Japanese Patent Application Laid-Open No. 11-222470
Patent Literature 6: Japanese Patent Application Laid-Open No. 2007-130194
Patent Literature 7: JP H 07-300513
Patent Literature 8: WO 2005/026224

### Non Patent Literature

Non Patent Literature 1: A. Henglein, Angew. Chem., 70, 461 (1955)
Non Patent Literature 2: Y. Ogiwara, et. al., Poym. Sci., Polym Letter Ed., 19, 457 (1981)

## Summary of Invention

### Problems to be Solved by the Invention

For the purpose of adsorbing cells, a technique of supporting cationic functional groups (e.g., a primary amino group, a secondary amino group, a tertiary amino group, and a quaternary ammonium group) or anionic functional groups (e.g., a sulfuric acid ester group, a sulfonic acid group, a carboxyl group, and a phosphoric acid ester group) to substrate surface in a balanced manner and performing the selective adsorption of cells has heretofore been known as a method for imparting selective cell adsorption performance to an adsorption substrate. However, for such a method, it is often difficult to exhibit the high cell selectivity of interest.

In contrast, a method using affinity ligands having specific affinity for proteins or sugar chains present on the cell surface of the cells of interest is effective for obtaining high cell-selective adsorptive properties. It is considered that, particularly, antibodies having an amino acid sequence with strong affinity for proteins or sugar chains on cell surface, antibody fragments (F(ab)', Fab, Fab', etc.) including the antigen-binding sites of the antibodies, and synthetic peptides having such an amino acid sequence or their modified peptides thereof are effective and that a method of supporting these on adsorption substrates by covalent bond is effective.

Furthermore, in the case of producing selective adsorbents, not limited to cell adsorbents, which selectively remove or collect the substances of interest from blood, safety in such a way that the leakage of ligands from the adsorbents is exceedingly small is also important. Specifically, it is necessary to substantially achieve the support of ligands by covalent bond to adsorbents with nonspecific adsorption unlikely to occur, and sufficiently reduce the physical noncovalent bond of the ligands.

The present invention has been done in consideration of the above situation, and an object to be attained is to provide: a substrate for ligand support which has such excellent biocompatibility that nonspecific interaction with biogenic substances such as proteins or blood cells is small and which is capable of being strongly bonded to ligands and exhibiting high adsorption performance based on specific interaction with the supported ligands; and a method for producing the same.

### Means for Solving the Problems

The present inventors have conducted diligent studies on a substrate which has excellent biocompatibility, which is capable of being strongly bonded to ligands whereby the risk of ligands' running out thereof is small, and which is capable of sufficiently inducing the performance of the supported ligands, and consequently completed the present invention by finding that a substrate for ligand support in which a copolymer made of CMB and a polymerizable monomer having an electrophilic functional group is bonded to the surface of a water-insoluble carrier attains the object.

Specifically, according to the present invention, the following invention is provided:

[1] A substrate for ligand support in which a copolymer represented by the following formula (1) is bonded to at least a surface of a water-insoluble carrier:

[Chemical Formula 1]

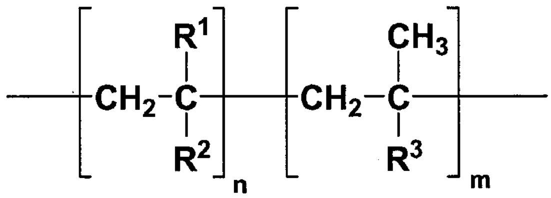

(1)

[in the formula (1), n and m represent a positive integer, a value of m / (n + m) is greater than or equal to 0.1 and less than or equal to 0.45, $R^1$ represents H or $CH_3$, $R^2$ represents an organic group including an electrophilic functional group, and $R^3$ represents

[Chemical Formula 2]

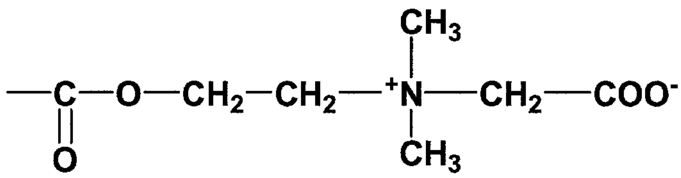

].

[2] A substrate for ligand support according to [1] in which a copolymer made of N-methacryloyloxyethyl-N,N-dimethylammonium-α-N-methylcarboxy betaine represented by the following formula (2) and a polymerizable monomer represented by the following formula (3) is bonded to at least a surface of a water-insoluble carrier, wherein a molar composition ratio of the N-methacryloyloxyethyl-N,N-dimethylammonium-α-N-methylcarboxy betaine unit to the copolymer is greater than or equal to 0.1 and less than or equal to 0.45:

[Chemical Formula 3]

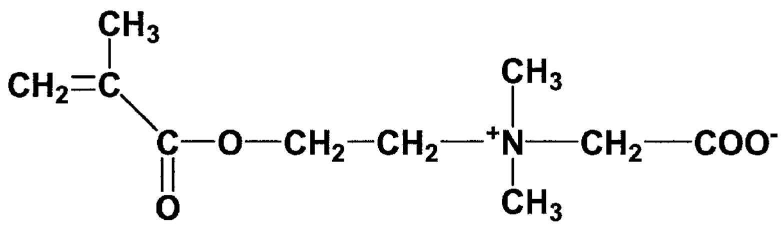

(2)

[Chemical Formula 4]

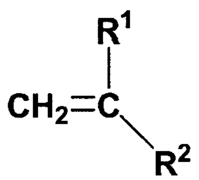

(3)

wherein in the formula (3), $R^1$ represents H or $CH_3$, and $R^2$ represents an organic group including an electrophilic

functional group.

[3] The substrate for ligand support according to [1] or [2], wherein the copolymer has an epoxy group.

[4] The substrate for ligand support according to any one of [1] to [3], wherein the coverage of the water-insoluble carrier surface with the copolymer is greater than or equal to 27%.

[5] The substrate for ligand support according to any one of [1] to [4], wherein the water-insoluble carrier is a porous membrane or particles.

[6] Use of a substrate comprising a copolymer represented by the following formula (1) bonded to at least a surface of a water-insoluble carrier for ligand support:

[Chemical Formula 1]

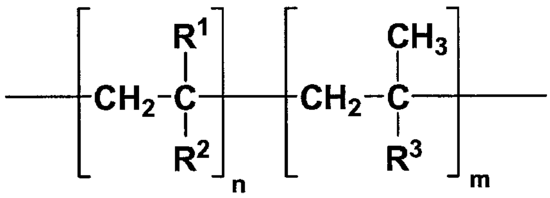

(1)

wherein in the formula (1), n and m represent a positive integer, a value of m / (n + m) is greater than or equal to 0.1 and less than or equal to 0.45, $R^1$ represents H or $CH_3$, $R^2$ represents an organic group including an electrophilic functional group, and $R^3$ represents

[Chemical Formula 2]

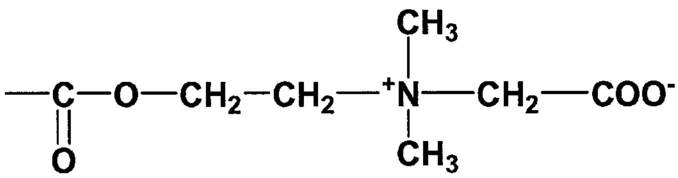

.

[7] The use of a copolymer according to [6], wherein the copolymer has an epoxy group.

[8] A method for producing a substrate for ligand support according to [1], comprising:

(i) a step of exposing a water-insoluble carrier to radiation; and

(ii) a step of dipping the water-insoluble carrier obtained in step (i) in a solution including N-methacryloyloxyethyl-N,N-dimethylammonium-α-N-methylcarboxy betaine represented by the following formula (2) and a polymerizable monomer represented by the following formula (3) to perform graft polymerization:

[Chemical Formula 3]

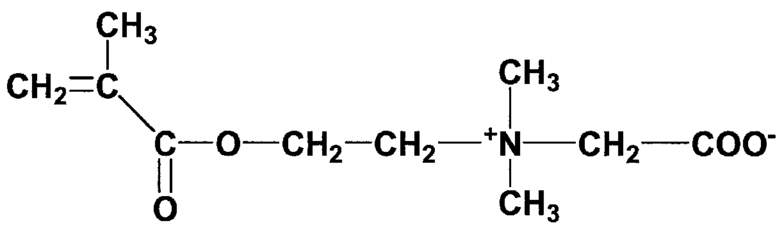

(2)

[Chemical Formula 4]

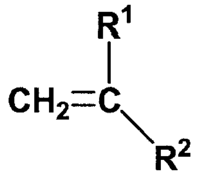

(3)

wherein in the formula (3), $R^1$ represents H or $CH_3$, and $R^2$ represents an organic group including an electrophilic functional group.

[9] The method for producing a substrate for ligand support according to [8], wherein the polymerizable monomer has an epoxy group.

[10] The method for producing a substrate for ligand support according to [8] or [9], wherein the water-insoluble carrier is a porous membrane or particles.

[11] An adsorbent in which a ligand selected from the group consisting of antibodies, proteins, peptides and low-molecular-weight compounds is bonded to the substrate for ligand support according to any one of [1] to [5].

[12] A blood treatment equipment in which the inside of a container equipped with an inlet for introducing blood to the inside and an outlet for discharging the blood to the outside is filled with the adsorbent according to [11].

**Effects of the Invention**

According to the present invention, there are provided: a substrate for ligand support which has such excellent biocompatibility that nonspecific interaction with biogenic substances such as proteins and blood cells is small and which is capable of being strongly bonded to ligands and exhibiting high adsorption performance based on specific interaction with the supported ligands; and a method for producing the same.

**Brief Description of Drawings**

Figure 1 is a schematic cross-sectional diagram of a substrate for ligand support according to one embodiment.
Figure 2 is a schematic cross-sectional diagram of an adsorbent according to one embodiment.
Figure 3 is a schematic cross-sectional diagram of a blood treatment equipment according to one embodiment.

**Embodiments for Carrying Out the Invention**

Hereinafter, the present invention will be described in detail. Embodiments described below are not intended to limit the present invention.

The substrate for ligand support of the present invention is characterized in that a copolymer represented by the following formula (1) is bonded to at least a surface of a water-insoluble carrier.

[Chemical Formula 1]

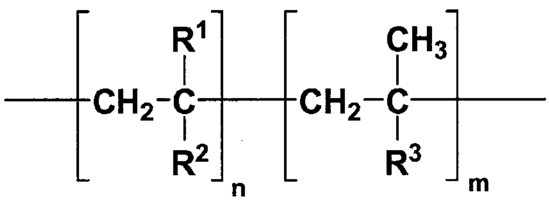

(1)

Here, in the formula (1), n and m represent a positive integer, and a value of m / (n + m) is greater than or equal to 0.1 and less than or equal to 0.45. Moreover, in the formula (1), $R^1$ represents H or $CH_3$, $R^2$ represents an organic group including an electrophilic functional group, and $R^3$ represents

[Chemical Formula 10]

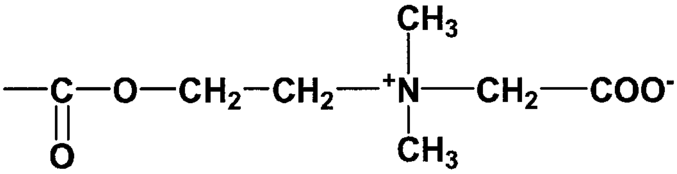

The copolymer represented by the formula (1) may be any of random copolymers, alternate copolymers, periodic copolymers, and block copolymers of a repeat unit in which the number of repeats is m and a repeat unit in which the number of repeats is n.

The copolymer represented by the formula (1) can be obtained by copolymerizing N-methacryloyloxyethyl-N,N-dimethylammonium-α-N-methylcarboxy betaine (CMB) represented by the formula (2) and a polymerizable monomer having an electrophilic functional group represented by the formula (3):

[Chemical Formula 11]

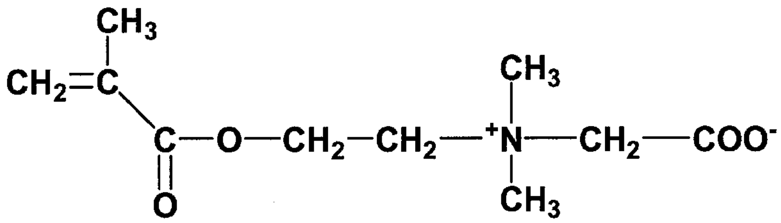

(2)

[Chemical Formula 12]

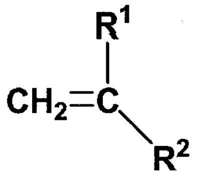

(3)

In the formula (3), $R^1$ represents H or $CH_3$, and $R^2$ represents an organic group including an electrophilic functional group.

Furthermore, the copolymer may be any of random copolymers, alternate copolymers, periodic copolymers, and block copolymers made of the compound represented by the formula (2) and the compound represented by the formula (3).

Moreover, in the formula (1), the repeat unit in which the number of repeats is m is a CMB-derived residue (hereinafter, referred to as a betaine group), and the repeat unit in which the number of repeats is n is a residue derived from the polymerizable monomer having an electrophilic functional group.

CMB represented by the formula (2) can be prepared easily with high purity by a method described in, for example, Japanese Patent Application Laid-Open No. 9-95474, Japanese Patent Application Laid-Open No. 9-95586, or Japanese Patent Application Laid-Open No. 11-222470.

In the present specification, the "electrophilic functional group" means a functional group that reacts in an electrophilic manner, and specific examples of the polymerizable monomer having an electrophilic functional group represented by the formula (3) include polymerizable monomers having an epoxy group, an isocyanate group, or an aldehyde group, or the like in the molecule ($R^2$ in the formula (1) and the formula (3)).

Examples of the polymerizable monomer having an epoxy group in the molecule can include, but not particularly limited to, glycidyl acrylate, glycidyl methacrylate, glycidyl acrylamide, allyl glycidyl ether, methacrylic glycidyl ether, glycidyl sorbate, glycidyl metaitaconate, ethyl glycidyl maleate, and glycidyl vinyl sulfonate. Examples of the polymerizable monomer having an isocyanate group in the molecule can include acryloyloxyethyl isocyanate, acryloyloxymethyl isocyanate, acryloyl isocyanate, methacryloyl isocyanate, and methacryloylethyl isocyanate. Furthermore, examples of the polymerizable monomer having an aldehyde group in the molecule can include cinnamic aldehyde, crotonaldehyde,

acrolein, and methacrolein. Among them, polymerizable monomers having an epoxy group in the molecule are preferable in terms of easiness of obtainment, cost, and easiness of handling, among which, particularly, glycidyl methacrylate is preferable.

What manner in which the copolymer is bonded to at least a surface of a water-insoluble carrier is not particularly limited and may be any of bonding manners such as covalent bond, ionic bond, and physical adsorption. Any generally known method such as a graft method or an insolubilization precipitation method can be used as a bonding method. Thus, a method of modifying surface by a generally known method of, for example, graft polymerizing or covalently bonding polymer compounds or monomers thereof using radiation or plasma, or the like (Japanese Patent Application Laid-Open No. 1-249063 and Japanese Patent Application Laid-Open No. 3-502094) is preferably used in the present invention.

In the case of using the radiation graft polymerization method, various generally known methods can be used. Although examples of ionizing radiation, for example, for introducing an active site onto the carrier include $\alpha$ rays, $\beta$ rays, $\gamma$ rays, accelerated electron beams, X rays, and UV rays, accelerated electron beams or $\gamma$ rays are preferable for actual use. Although the dose of the accelerated electron beams or $\gamma$ rays can be changed arbitrarily according to the properties of the carrier, the properties of the polymerizable monomer, the amount of binding, etc., 10 kGy to 200 kGy is preferable. Although any of a simultaneous irradiation method of delivering radiation under the coexistence of the carrier and the polymerizable monomer and a preirradiation method of delivering radiation only to the carrier in advance and then contacting the polymerizable monomer with the carrier may be used as a method for graft-polymerizing the carrier and the polymerizable monomer according to the present invention, the preirradiation method is preferable because of being unlikely to cause side reaction other than graft polymerization. The reaction between the polymerizable monomer and the carrier can be performed in ranges in which the polymerizable monomer concentration is usually 1% by mass or more and 20% by mass or less and the reaction temperature is 5°C or more and 40°C or less, but is not limited to the ranges and may be set appropriately. A solvent for the reaction between the polymerizable monomer and the carrier is not used, or solvents such as water, methanol, ethanol, other alcohols, or acetone or mixed solvents thereof can be used as long as the polymerizable monomer can be dissolved or dispersed. Moreover, in the case of using the radiation graft polymerization method, the graft ratio (G) of the copolymer to the carrier is preferably 5% or more and 300% or less, more preferably 20% or more and 200% or less, particularly preferably 50% or more and 150% or less. If the graft ratio is less than 5%, the covering of the carrier surface with graft chains tends to become insufficient, and there may be a possiblity that the modification of the carrier surface becomes insufficient or that the amount of ligands supported becomes insufficient. Moreover, if the graft ratio exceeds 300%, there may be a possibility that the physical properties of the carrier itself are lost and this is not preferable in terms of the design of an adsorbent.

Furthermore, the graft ratio (G) described here is a value represented by the following formula (4):

$$G\ (\%) = [(\text{Carrier weight after grafting} - \text{Carrier weight before grafting}) / (\text{Carrier weight before grafting})] \times 100 \qquad (4)$$

After the polymerization reaction, it is preferred to sufficiently wash off with an appropriately solvent, excessive monomers or ungrafted polymers formed by chain transfer reaction.

It is preferred that the molar composition ratio of the N-methacryloyloxyethyl-N,N-dimethylammonium-$\alpha$-N-methylcarboxy betaine (CMB) unit to the copolymer of the present invention should be greater than or equal to 0.1 and less than or equal to 0.45. If the molar composition ratio of the betaine group is less than 0.1, the property of biocompatibility is not exhibited. Moreover, if the molar composition ratio of the betaine group exceeds 0.45, the property of biocompatibility is sufficiently exhibited, but inevitably the content of the electrophilic functional group becomes low and the support of ligands is not sufficiently performed; thus this is not preferable. The amount of each polymerizable monomer mixed during the reaction forming the copolymer depends on a selected solvent, etc., and can therefore be selected appropriately.

The coverage of the water-insoluble carrier surface with the copolymer is preferably greater than or equal to 27%, more preferably greater than or equal to 27% and less than or equal to 75%. If the coverage of the carrier surface is less than 27%, the surface properties of the carrier tend to easily remain and there may be a possibility that nonspecific adsorption increases or that the support of ligands is not sufficiently performed.

Examples of a material forming the insoluble carrier used in the present invention include, but not particularly limited to, organic polymer compounds such as natural polymers, synthetic polymers, and regenerated polymers, inorganic compounds typified by glass and metal, and organic/inorganic hybrid compounds.

Organic polymer materials are particularly preferable in terms of workability, and examples thereof include polyalkylene terephthalates, polycarbonates, polyurethanes, poly(meth)acrylic acid esters, polyacrylonitrile, polyvinyl alcohol, ethylene/vinyl alcohol copolymers (Eval), ethylene/vinyl acetate copolymers, polyvinylacetal, polystyrene,

polysulfones, cellulose and cellulose derivatives, polyphenylene ethers, polyethylene, polypropylene, polyvinyl fluoride, polyvinyl chloride, polyvinylidene fluoride, and copolymers of monomers constituting these, and further, alloys or blends of one type or two or more types of the above polymers.

Although all of those well known as carriers for medical adsorbents can be used as the water-insoluble carrier, a porous membrane or particles are preferable. Porous particles, a nonwoven fabric, or a hollow fiber membrane is most preferable in terms of the flowability of body fluids during extracorporeal circulation. In the case of intending cell adsorption, a nonwoven fabric or particles are preferable.

Examples of the porous membrane include nonwoven fabric, woven fabrics, knitted fabrics, and meshes produced using fibrous substances (solid fibers or hollow fibers) obtained from these materials. Moreover, a sheet-shaped membrane (flat membrane) or hollow fiber membrane that can be obtained by a foaming method, a phase separation method (heat-induced phase separation method or wet phase separation method), a drawing method, a sintering method, or the like from a thermally melted state of organic polymer materials or inorganic polymer materials, a solution state thereof dissolved in a solvent, a state thereof plasticized using a plasticizer, or the like can also be used.

For the porous membrane, any structure having continuous holes may be used and is not particularly limited as long as the components of interest are adsorbed by contact with affinity ligands present in at least the surface portions of the continuous holes. The continuous holes refer to holes that continue from one membrane surface of the supporting porous membrane through the other membrane surface, and the membrane surface shapes of the holes or the structure inside the membranes can be any form as long as liquids can pass through the continuous holes.

Particularly, examples of one that is preferred in the case where the adsorption components of interest are cells include nonwoven fabrics, woven fabrics, knitted fabrics, and meshes produced from various fibrous substances, from the viewpoint of the permeability of cell suspensions and cell-trapping properties, and, particularly, a nonwoven fabric is a porous membrane preferably used. In the case of a nonwoven fabric shape, its average fiber diameter is preferably 0.1 μm to 50 μm. Furthermore, it is preferred that the average fiber diameter should be 0.5 μm to 40 μm, it is more preferred to be 1 μm to 30 μm, it is particularly preferred to be 1 μm to 20 μm, and it is most preferred to be 2 μm to 10 μm. If the average fiber diameter is less than 0.1 μm, the mechanical strength of the substrate for ligand support or a cell-selective adsorbent tends to decrease. Moreover, if the average fiber diameter exceeds 50 μm, an adsorption surface area in the case of being a cell-selective adsorbent becomes small and cell-trapping properties tend to decrease.

The particles used in the present invention are nonporous or porous, and particle sizes thereof can be selected for the porous ones according to the adsorption components of interest. Although ones in which the average particle size is greater than or equal to 25 μm and less than or equal to 2500 μm can be used, ones in which the average particle size is greater than or equal to 50 μm and less than or equal to 1500 μm are preferable in consideration of specific surface areas thereof (adsorption ability as an adsorbent) and the flowability of body fluids.

Figure 1 is a schematic cross-sectional diagram of a substrate for ligand support according to one embodiment. A substrate 100 for ligand support comprises a water-insoluble carrier 1 and a copolymer 2 represented by the above formula (1) bonded to at least the surface of the water-insoluble carrier 1. The copolymer 2 is bonded to the water-insoluble carrier 1 via a bonding moiety 10 and has an $R^3$ group 20 and an $R^2$ group 30 on the surface of the substrate 100 for ligand support. Here, the $R^3$ group 20 and the $R^2$ group 30 have the same meaning as the $R^3$ group and the $R^2$ group in the above formula (1). Specifically, the $R^3$ group 20 represents

[Chemical Formula 2]

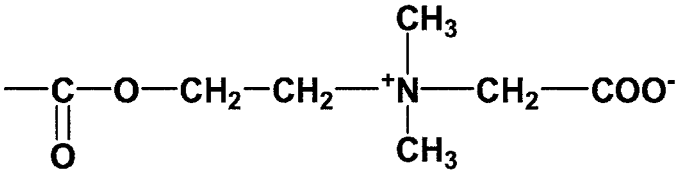

and the $R^2$ group 30 represents an electrophilic functional group. Furthermore, the bonding moiety 10 is a moiety mainly corresponding to the main chain of the copolymer 2.

In the adsorbent of the present invention, ligands supported on the substrate for ligand support can be affinity ligands having selective affinity for particular blood components. The affinity ligands are not particularly limited as long as being ones chemically bonded to the electrophilic functional group. Use of low-molecular-weight compounds in which the molecular weight is less than or equal to approximately 500 Da, proteins, antibodies or chimeric antibodies, $F(ab')_2$, Fab, or Fab' having an amino acid sequence capable of forming a complementarity determining region in the variable region of a heavy chain or light chain possessed by the antibodies having exceedingly high affinity for the target components in order to elicit excellent adsorption ability, and other peptides or modified peptide-type ligands thereof is

preferable.

Figure 2 is a schematic cross-sectional diagram of an adsorbent according to one embodiment. An adsorbent 110 is one in which the $R^2$ group 30 of the substrate 100 for ligand support is chemically bonded to a ligand 40. Those described above can be used as the ligand 40.

The blood treatment equipment of the present invention is one in which the inside of a container equipped with an inlet for introducing blood to the inside and an outlet for discharging the blood from the inside to the outside is filled with the above adsorbent. The above adsorbent can selectively adsorb the target components and can therefore be used preferably, for example, for the purpose of removing particular components from blood. Specifically, examples thereof include blood component adsorbers for direct blood perfusion and specific cell adsorbers.

Figure 3 is a schematic cross-sectional diagram of a blood treatment equipment according to one embodiment. A blood treatment equipment 200 comprises a container 50 and a plurality of adsorbents 110 with which the inside of the container 50 is filled. Header caps 60a and 60b are provided at both ends of the container 50. The header cap 60a serves as the inlet for introducing blood to the inside (introduction port), and the header cap 60b serves as the outlet for discharging the blood to the outside (discharge port). Blood that has flowed to the inside of the blood treatment equipment 200 in the direction of the arrow F from the introduction port of the header cap 60a comes in contact with the adsorbents 110, whereby components in the blood interacting with the ligand 40 are adsorbed. As a result, the above components have already been removed from the blood when the blood flows out of the discharge port of the header cap 60b.

The substrate according to the present invention in which a copolymer represented by the above formula (1) is bonded to at least a surface of a water-insoluble carrier can be used for supporting ligands used in an adsorbent or blood treatment equipment for removing particular components (components specifically binding to the ligands) from blood.

**Examples**

Although the constitution and effects of the present invention will be specifically described below with reference to Examples, any of them are not intended to limit the present invention.

[Example 1]

(A) Method

(1) γ ray irradiation

A 0.108 $m^2$ nonwoven fabric (average fiber diameter: 3.8 μm, weight: 80 $g/m^2$) made of polypropylene was enclosed as a carrier together with a deoxidizer in an oxygen-low-permeable bag to sufficiently remove oxygen and then irradiated with y rays of 25 kGy at -78°C.

(2) Graft reaction

10.8 g of N-methacryloyloxyethyl-N,N-dimethylammonium-α-N-methylcarboxy betaine and 26.3 mL of glycidyl methacrylate (GMA) were dissolved in 500 mL of methanol and purged with nitrogen at 40°C for 60 minutes.

The above nonwoven fabric was quickly placed in a pressure-resistant glass container, and after pressure reduction, the above solution was brought therein and reacted at 40°C for 1 hour. After the reaction, the nonwoven fabric taken out thereof was washed with dimethylformamide and methanol and vacuum dried at 40°C to obtain a nonwoven fabric for ligand support (substrate for ligand support).

(3) Preparation of cell adsorption filter (adsorbent)

The nonwoven fabric for ligand support obtained above was cut into a round shape of 0.68 cm in diameter (hereinafter, referred to a "round nonwoven fabric substrate"). Next, four obtained round nonwoven fabric substrates were dipped at 37°C for 16 hours in 400 μL of calcium- and magnesium-free phosphate-buffered saline (hereinafter, referred to as "PBS (-)") in which anti-human CD4 monoclonal antibodies (16 μg) and ammonium sulfate (26 mg) were dissolved, to support the monoclonal antibodies to the round nonwoven fabric substrates. Then, this round nonwoven fabric substrate on which the monoclonal antibodies were supported (hereinafter, referred to as an "antibody-supported round nonwoven fabric") was washed with 2 ml of PBS (-). Next, the antibody-supported round nonwoven fabric was dipped in a 0.2% polyoxyethylene sorbitan monolaurate/PBS (-) solution (hereinafter, referred to as a "Tween 20 solution") at room temperature for 2.5 hours to perform blocking. Then, the antibody-supported round nonwoven fabric was washed with 2 ml of PBS (-) to obtain a cell adsorption filter.

(4) Washing of cell adsorption filter

Four cell adsorption filters were dipped in 2% sodium dodecyl sulfate/PBS (-) (hereinafter, referred to as an "SDS solution") at 95°C for 10 minutes. This was repeated three times, and the cell adsorption filters were then washed with 2 ml of PBS (-).

(5) Preparation of cell adsorber (blood treatment equipment)

A container of 1 ml in volume having an inlet and an outlet was filled with four cell adsorption filters obtained above and a PBS (-) solution as a filling fluid to prepare a cell adsorber.

(6) Cell-adsorptive properties of cell adsorber

From the inlet of the cell adsorber obtained above, 8 ml of ACD-A-supplemented human fresh blood (blood:ACD-A = 8:1) was fed at a flow rate of 0.2 ml/min with a syringe pump. Fractionation was performed so that of a solution after cell adsorption collected from the outlet of the cell adsorber, a 4 mL aliquot in the first half and a 4 mL aliquot in the latter half became fraction 1 (hereinafter, referred to as "F1") and fraction 2 (hereinafter, referred to as "F2"), respectively. Then, the solution after cell adsorption was collected from the outlet of the cell adsorber.

(B) Results

(1) Surface analysis by X-ray photoelectron spectroscopy (XPS)

The surface of the nonwoven fabric for ligand support obtained above was analyzed by X-ray photoelectron spectroscopy (XPS ESCA). When the respective relative element concentrations of carbon, oxygen, and nitrogen obtained by XPS are defined as $C_1$, $C_2$, and $C_3$, respectively, the relative molar concentration (X) of CMB present on the substrate surface, the relative molar concentration (Y) of GMA, and the relative molar concentration (Z) of the carrier-constituting polymer are represented by the following formulas (5) to (7), respectively:

$$X = x / (x + y + z) \qquad (5)$$

$$Y = y / (x + y + z) \qquad (6)$$

$$Z = z / (x + y + z) \qquad (7)$$

However, $x = C_3$, $y = (C_2 - 4x) / 3$, and $z = \{C_1 - (10x + 7y)\} / A$, wherein A is the unit carbon composition of the carrier-constituting polymer, for example, A = 2 in the case of polyethylene or A = 3 in the case of polypropylene.

From the relative molar concentrations obtained above, the molar composition ratio R of CMB unit in the copolymer is represented by the following formula (8):

$$R = m / (n + m) = X / (X + Y) \qquad (8)$$

Moreover, when the unit molecular weight of the CMB polymer, the unit molecular weight of the GMA polymer, and the unit molecular weight of the carrier-constituting polymer are defined as $M_X$, $M_Y$, and $M_Z$, respectively, the coverage S (%) is represented by the following formula (9):

$$S = 100\{1 - ZM_Z / (XM_X + YM_Y + ZM_Z)\} \qquad (9)$$

As a result of calculating the molar composition ratio R of CMB unit and the coverage S based on these calculation formulas, the molar composition ratio R of CMB unit was 0.1, and the coverage S was 75%.

(2) Specific surface area measurement

The specific surface area of the nonwoven fabric for ligand support obtained above was measured by a multipoint method based on the BET method using an automatic specific surface area/pore distribution measurement apparatus (MICRIMERITICS TRISTAR 3000 manufactured by Shimadzu Corp.). As a result, the specific surface area was 0.60 $m^2/g$.

(3) Determination of the amount of antibodies supported

The amount of anti-human CD4 monoclonal antibodies supported on the cell adsorption filter obtained above was determined by performing absorbance measurement with a microplate spectrophotometer (SPECTRA MAX340PC, analysis software SOFT max PRO, manufactured by Molecular Devices, LLC.) using a BCA protein quantification reagent (Micro BCA (registered trademark) Protein Assay Reagent Kit 23235 manufactured by Pierce Biotechnology, Inc.). As a result, 10.0 mg and 8.0 mg of the antibodies were supported on the cell adsorption filters (four) before washing and the cell adsorption filters after SDS washing, respectively. (4) Adsorption rates of CD4-positive cell and platelet

The number of CD4-positive cells in ACD-A-supplemented human fresh blood before cell adsorption and the solutions after cell adsorption (F1 and F2) was determined by flow cytometry using a flow cytometer (FACSCALIBUR manufactured by Becton, Dickinson and Company), and the adsorption rate of the cells to the cell adsorber was calculated. As a result, the adsorption rate of the CD4-positive cells was 93.2% (F1) and 60.1% (F2). Moreover, the adsorption rate of platelets was 35.6% (8 mL-average).

[Example 2]

Graft reaction was performed by the same method as in Example 1 using as a reaction solution, one in which 21.5 g of N-methacryloyloxyethyl-N,N-dimethylammonium-α-N-methylcarboxy betaine and 26.3 mL of glycidyl methacrylate were dissolved in 500 mL of methanol, to obtain a nonwoven fabric for ligand support. As a result of surface analysis by XPS, the molar composition ratio R of CMB unit was 0.26, and the coverage S was 54%. Moreover, the specific surface area was determined to be 0.50 $m^2/g$. As a result of preparing a cell adsorption filter from the nonwoven fabric and measuring the amount of antibodies supported thereon, 11.6 mg and 8.4 mg of the antibodies were supported on the cell adsorption filters before washing and the cell adsorption filters after SDS washing, respectively. As a result of preparing a cell adsorber from the cell adsorption filter and determining the adsorption rate of cells, the adsorption rate of the CD4-positive cells was 99.7% (F1) and 89.0% (F2). Moreover, the adsorption rate of platelets was 21.4% (8 mL-average).

[Example 3]

Graft reaction was performed by the same method as in Example 1 using as a reaction solution, one in which 43.1 g of N-methacryloyloxyethyl-N,N-dimethylammonium-α-N-methylcarboxy betaine and 26.3 mL of glycidyl methacrylate were dissolved in 500 mL of methanol, to obtain a nonwoven fabric for ligand support. As a result of surface analysis by XPS, the molar composition ratio R of CMB unit was 0.32, and the coverage S was 54%. Moreover, the specific surface area was determined to be 0.51 $m^2/g$. As a result of preparing a cell adsorption filter from the nonwoven fabric and measuring the amount of antibodies supported thereon, 9.6 mg and 8.8 mg of the antibodies were supported on the cell adsorption filters before washing and the cell adsorption filters after SDS washing, respectively. As a result of preparing a cell adsorber from the cell adsorption filter and determining the adsorption rate of cells, the adsorption rate of the CD4-positive cells was 95.1% (F1) and 86.0% (F2). Moreover, the adsorption rate of platelets was 12.4% (8 mL-average).

[Example 4]

Graft reaction was performed by the same method as in Example 1 using as a reaction solution, one in which 68.9 g of N-methacryloyloxyethyl-N,N-dimethylammonium-α-N-methylcarboxy betaine and 21.1 mL of glycidyl methacrylate were dissolved in 400 mL of methanol, to obtain a nonwoven fabric for ligand support. As a result of surface analysis by XPS, the molar composition ratio R of CMB unit was 0.45, and the coverage S was 55%. Moreover, the specific surface area was determined to be 0.37 $m^2/g$. As a result of preparing a cell adsorption filter from the nonwoven fabric and measuring the amount of antibodies supported thereon, 9.2 mg and 7.8 mg of the antibodies were supported on the cell adsorption filters before washing and the cell adsorption filters after SDS washing, respectively. As a result of preparing a cell adsorber from the cell adsorption filter and determining the adsorption rate of cells, the adsorption rate of the CD4-positive cells was 92.5% (F1) and 52.4% (F2). Moreover, the adsorption rate of platelets was 4.6% (8 mL-average).

[Example 5]

Particles of 320 μm in average particle size made of polyethylene were enclosed together with a deoxidizer in an oxygen-low-permeable bag to sufficiently remove oxygen and then irradiated with γ rays of 25 kGy at -78°C.

Graft reaction was performed by the same method as in Example 1 using as a reaction solution, one in which 21.5 g of N-methacryloyloxyethyl-N,N-dimethylammonium-α-N-methylcarboxy betaine and 26.3 mL of glycidyl methacrylate were dissolved in 500 mL of methanol, to obtain particles for ligand support (substrate for ligand support). As a result of surface analysis by XPS, the molar composition ratio R of CMB unit was 0.25, and the coverage S was 44%. Moreover, the specific surface area was determined to be 0.027 $m^2/g$. As a result of preparing a cell adsorption filter from 360 μL of the particles for ligand support and measuring the amount of antibodies supported thereon, 15.2 mg and 10.1 mg of the antibodies were supported on the cell adsorption filters before washing and the cell adsorption filters after SDS washing, respectively. As a result of preparing a cell adsorber from the cell adsorption filter and determining the adsorption rate of cells, the adsorption rate of the CD4-positive cells was 84.8% (F1) and 73.5% (F2). Moreover, the adsorption rate of platelets was 2.0% (8 mL-average).

[Example 6]

Graft reaction was performed by the same method as in Example 1 using as a reaction solution, one in which 10.8 g of N-methacryloyloxyethyl-N,N-dimethylammonium-α-N-methylcarboxy betaine and 13.2 mL of glycidyl methacrylate were dissolved in 500 mL of methanol, to obtain a nonwoven fabric for ligand support. As a result of surface analysis by XPS, the molar composition ratio R of CMB unit was 0.27, and the coverage S was 27%. Moreover, the specific surface area was determined to be 0.56 $m^2/g$. As a result of preparing a cell adsorption filter from the nonwoven fabric and measuring the amount of antibodies supported thereon, 11.0 mg and 8.2 mg of the antibodies were supported on the cell adsorption filters before washing and the cell adsorption filters after SDS washing, respectively. As a result of preparing a cell adsorber from the cell adsorption filter and determining the adsorption rate of cells, the adsorption rate of the CD4-positive cells was 99.3% (F1) and 87.1% (F2). Moreover, the adsorption rate of platelets was 21.0% (8 mL-average).

[Comparative Example 1]

Graft reaction was performed by the same method as in Example 1 using as a reaction solution, one in which 26.3 mL of glycidyl methacrylate was dissolved in 500 mL of methanol, to obtain a nonwoven fabric for ligand support. As a result of surface analysis by XPS, the molar composition ratio R of CMB unit was 0, and the coverage S was 64%. Moreover, the specific surface area was determined to be 0.51 $m^2/g$. As a result of preparing a cell adsorption filter from the nonwoven fabric and measuring the amount of antibodies supported thereon, 10.6 mg and 7.5 mg of the antibodies were supported on the cell adsorption filters before washing and the cell adsorption filters after SDS washing, respectively. As a result of preparing a cell adsorber from the cell adsorption filter and determining the adsorption rate of cells, the adsorption rate of the CD4-positive cells was 91.9% (F1) and 34.4% (F2). Moreover, the adsorption rate of platelets was 81.2% (8 mL-average).

[Comparative Example 2]

Graft reaction was performed by the same method as in Example 1 using as a reaction solution, one in which 103.3 g of N-methacryloyloxyethyl-N,N-dimethylammonium-α-N-methylcarboxy betaine and 15.8 mL of glycidyl methacrylate were dissolved in 350 mL of methanol, to obtain a nonwoven fabric for ligand support. As a result of surface analysis by XPS, the molar composition ratio R of CMB unit was 0.56, and the coverage S was 56%. Moreover, the specific surface area was determined to be 0.36 $m^2/g$. As a result of preparing a cell adsorption filter from the nonwoven fabric and measuring the amount of antibodies supported thereon, 0.5 mg and 0.1 mg of the antibodies were supported on the cell adsorption filters before washing and the cell adsorption filters after SDS washing, respectively. As a result of preparing a cell adsorber from the cell adsorption filter and determining the adsorption rate of cells, the adsorption rate of the CD4-positive cells was 23.2% (F1) and 0.2% (F2). Moreover, the adsorption rate of platelets was 5.9% (8 mL-average).

These results were summarized in Table 1 and Table 2.

[Table 1]

| Example | Substrate for ligand support | | | Surface analysis | | |
|---|---|---|---|---|---|---|
| | Shape | Monomer concentration (mol/L) | | XPS | | Specific surface area ($m^2/g$) |
| | | GMA | CMB | CMB Molar composition ratio R m/(n+m) | Coverage S (%) | |
| Example 1 | Nonwoven fabric | 0.38 | 0.10 | 0.10 | 75 | 0.60 |
| Example 2 | Nonwoven fabric | 0.38 | 0.19 | 0.26 | 54 | 0.50 |
| Example 3 | Nonwoven fabric | 0.38 | 0.38 | 0.32 | 54 | 0.51 |
| Example 4 | Nonwoven fabric | 0.38 | 0.76 | 0.45 | 55 | 0.37 |
| Example 5 | Particles | 0.38 | 0.19 | 0.27 | 44 | 0.027 |
| Example 6 | Nonwoven fabric | 0.38 | 0.19 | 0.24 | 27 | 0.56 |
| Comparative Example 1 | Nonwoven fabric | 0.33 | 0 | 0 | 64 | 0.51 |
| Comparative Example 2 | Nonwoven fabric | 0.38 | 1.31 | 0.56 | 56 | 0.36 |

[Table 2]

| Example | Cell adsorption filter | | | Cell adsorber | | |
|---|---|---|---|---|---|---|
| | Ligand name | Amount of antibodies supported (μg) | | CD4-positive cell adsorption rate (%) | | Platelet adsorption rate (8 mL-average) |
| | | Before SDS washing | After SDS washing | F1 | F2 | |
| Example 1 | Anti-CD4 antibody | 10.0 | 8.0 | 93.2 | 60.1 | 35.6 |
| Example 2 | Anti-CD4 antibody | 11.6 | 8.4 | 99.7 | 89.0 | 21.4 |
| Example 3 | Anti-CD4 antibody | 9.6 | 8.8 | 95.1 | 86.0 | 12.4 |
| Example 4 | Anti-CD4 antibody | 9.2 | 7.8 | 92.5 | 52.4 | 4.6 |
| Example 5 | Anti-CD4 antibody | 15.2 | 10.1 | 84.8 | 73.5 | 2.0 |
| Example 6 | Anti-CD4 antibody | 11.0 | 8.2 | 99.3 | 87.1 | 21.0 |
| Comparative Example 1 | Anti-CD4 antibody | 10.0 | 7.5 | 91.9 | 34.4 | 81.2 |
| Comparative Example 2 | Anti-CD4 antibody | 0.5 | 0.1 | 23.2 | 0.2 | 5.9 |

## Reference Signs List

1 ... Water-insoluble carrier, 2 ... Copolymer, 10 ... Bonding moiety, 20 ... $R^3$ group, 30 ... $R^2$ group (electrophilic functional group), 40 ... Ligand, 50 ... Container, 60a and 60b ... Header cap, F ... Flowing direction of blood, 100 ... Substrate for ligand support, 110 ... Adsorbent, 200 ... Blood treatment equipment.

## Claims

1. A substrate for ligand support in which a copolymer represented by the following formula (1) is bonded to at least a surface of a water-insoluble carrier:

[Chemical Formula 1]

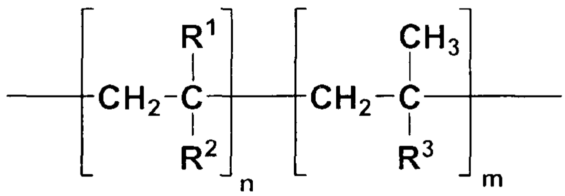

(1)

wherein in the formula (1), n and m represent a positive integer, a value of m/(n + m) is greater than or equal to 0.1 and less than or equal to 0.45, $R^1$ represents H or $CH_3$, $R^2$ represents an organic group including an electrophilic functional group, and $R^3$ represents

[Chemical Formula 2]

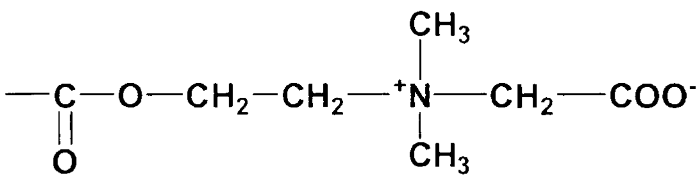

2. The substrate for ligand support according to claim 1 in which a copolymer made of N-methacryloyloxyethyl-N,N-dimethylammonium-α-N-methylcarboxybetaine represented by the following formula (2) and a polymerizable monomer represented by the following formula (3) is bonded to at least a surface of a water-insoluble carrier, wherein a molar composition ratio of the N-methacryloyloxyethyl-N,N-dimethylammonium-α-N-methylcarboxybetaine unit to the copolymer is greater than or equal to 0.1 and less than or equal to 0.45:

[Chemical Formula 3]

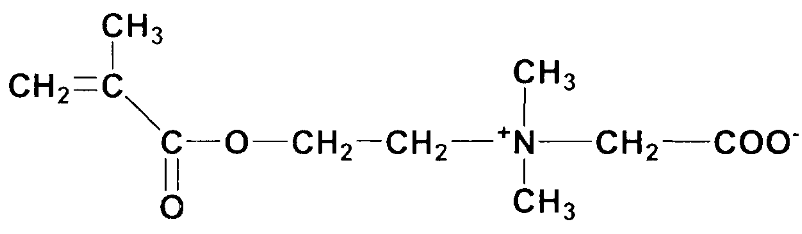

(2)

[Chemical Formula 4]

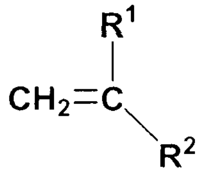

(3)

wherein in the formula (3), $R^1$ represents H or $CH_3$, and $R^2$ represents an organic group including an electrophilic functional group.

3. The substrate for ligand support according to claim 1 or 2, wherein the copolymer has an epoxy group.

4. The substrate for ligand support according to any one of claims 1 to 3, wherein the coverage of the water-insoluble carrier surface with the copolymer is greater than or equal to 27%.

5. The substrate for ligand support according to any one of claims 1 to 4, wherein the water-insoluble carrier is a porous membrane or particles.

6. Use of a substrate comprising a copolymer represented by the following formula (1) bonded to at least a surface of a water-insoluble carrier for ligand support:

[Chemical Formula 1]

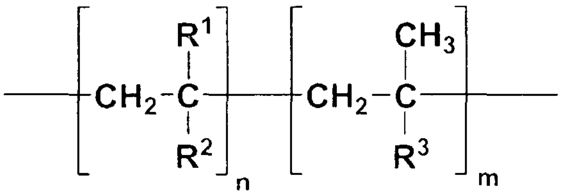

(1)

wherein in the formula (1), n and m represent a positive integer, a value of m / (n + m) is greater than or equal to 0.1 and less than or equal to 0.45, $R^1$ represents H or $CH_3$, $R^2$ represents an organic group including an electrophilic functional group, and $R^3$ represents

[Chemical Formula 2]

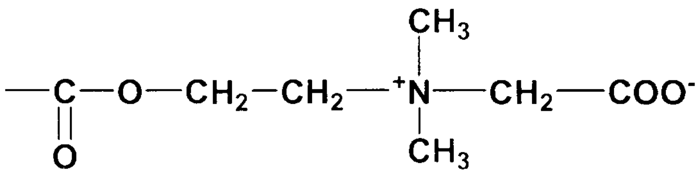

7. The use of a copolymer according to claim 6, wherein the copolymer has an epoxy group.

8. A method for producing a substrate for ligand support according to claim 1, comprising:

(i) a step of exposing a water-insoluble carrier to radiation; and
(ii) a step of dipping the water-insoluble carrier obtained in step (i) in a solution including N-methacryloyloxyethyl-N,N-dimethylammonium-α-N-methylcarboxybetaine represented by the following formula (2) and a polymerizable monomer represented by the following formula (3) to perform graft polymerization:

[Chemical Formula 3]

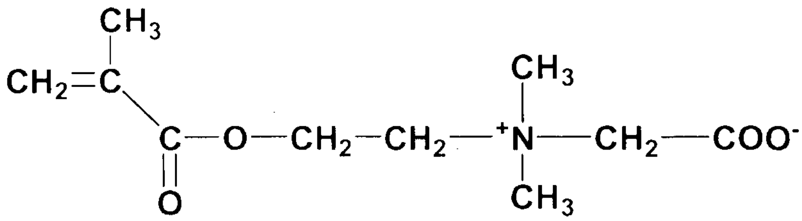

(2)

[Chemical Formula 4]

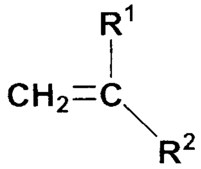

(3)

wherein in the formula (3), $R^1$ represents H or $CH_3$, and $R^2$ represents an organic group including an electrophilic functional group.

9. The method for producing a substrate for ligand support according to claim 8, wherein the polymerizable monomer has an epoxy group.

10. The method for producing a substrate for ligand support according to claim 8 or 9, wherein the water-insoluble carrier is a porous membrane or particles.

11. An adsorbent in which a ligand selected from the group consisting of antibodies, proteins, peptides and low-molecular-weight compounds is bonded to the substrate for ligand support according to any one of claims 1 to 5.

12. A blood treatment equipment in which the inside of a container equipped with an inlet for introducing blood to the inside and an outlet for discharging the blood to the outside is filled with the adsorbent according to claim 11.

**Patentansprüche**

1. Substrat als Ligandenträger, bei dem ein Copolymer, das durch die folgende Formel (1) dargestellt wird, an wenigstens eine Fläche eines wasserunlöslichen Trägers gebunden ist:

[chemische Formel 1]

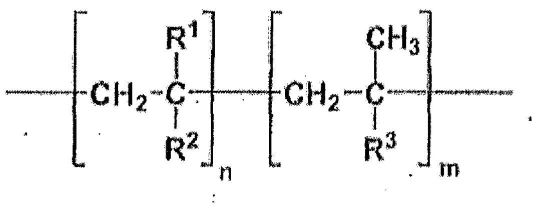

(1)

wobei in der Formel (1) n und m für eine positive ganze Zahl stehen, der Wert von m/(n + m) größer oder gleich 0,1 und kleiner oder gleich 0,45 ist, $R^1$ für H oder $CH_3$ steht, $R^2$ für eine organische Gruppe, die eine elektrophile funktionelle Gruppe umfasst, steht und $R^3$ für

[chemische Formel 2]

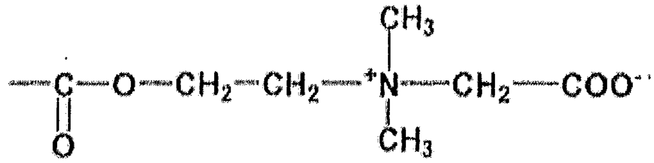

steht.

2. Substrat als Ligandenträger gemäß Anspruch 1, wobei ein Copolymer, das aus N-Methacryloyloxyethyl-N,N-dimethylammonium-$\alpha$-N-methylcarboxy-betain, das durch die folgende Formel (2) dargestellt wird, und einem polymerisierbaren Monomer, das durch die folgende Formel (3) dargestellt wird, hergestellt ist, an wenigstens eine Fläche eines wasserunlöslichen Trägers gebunden ist, wobei das Stoffmengenzusammensetzungsverhältnis der N-Methacryloyloxyethyl-N,N-dimethylammonium-$\alpha$-N-methylcarboxybetain-Einheit zu dem Copolymer größer oder gleich 0,1 und kleiner oder gleich 0,45 ist:

[chemische Formel 3]

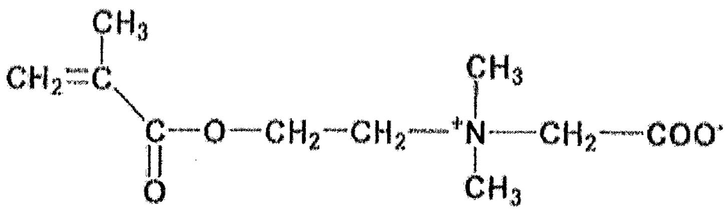

(2)

[chemische Formel 4]

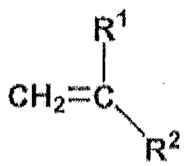

(3),

wobei in der Formel (3) $R^1$ für H oder $CH_3$ steht und $R^2$ für eine organische Gruppe, die eine elektrophile funktionelle Gruppe umfasst, steht.

3. Substrat als Ligandenträger gemäß Anspruch 1 oder 2, wobei das Copolymer eine Epoxidgruppe aufweist.

4. Substrat als Ligandenträger gemäß einem der Ansprüche 1 bis 3, wobei die Bedeckungsrate der Fläche des wasserunlöslichen Trägers mit dem Copolymer größer oder gleich 27% ist.

5. Substrat als Ligandenträger gemäß einem der Ansprüche 1 bis 4, wobei es sich bei dem wasserunlöslichen Träger um eine poröse Membran oder um Teilchen handelt.

6. Verwendung eines Substrats, das ein durch die folgende Formel (1) dargestelltes Copolymer, welches an wenigstens eine Fläche eines wasserunlöslichen Trägers gebunden ist, umfasst, als Ligandenträger:

[chemische Formel 1]

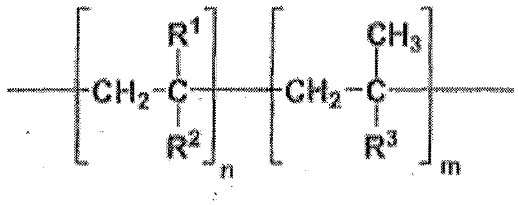

(1)

wobei in der Formel (1) n und m für eine positive ganze Zahl stehen, der Wert von m/(n + m) größer oder gleich 0,1 und kleiner oder gleich 0,45 ist, $R^1$ für H oder $CH_3$ steht, $R^2$ für eine organische Gruppe, die eine elektrophile funktionelle Gruppe umfasst, steht und $R^3$ für

[chemische Formel 2]

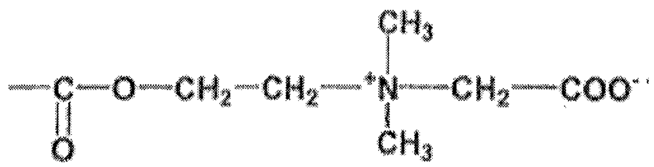

steht.

**7.** Verwendung eines Copolymers gemäß Anspruch 6, wobei das Copolymer eine Epoxidgruppe aufweist.

**8.** Verfahren zur Herstellung eines Substrats als Ligandenträger gemäß Anspruch 1, umfassend:

(i) einen Schritt des Einwirkenlassens von Strahlung auf einen wasserunlöslichen Träger; und
(ii) einen Schritt des Eintauchens des in Schritt (i) erhaltenen wasserunlöslichen Trägers in eine Lösung, die N-Methacryloyloxyethyl-N,N-dimethylammonium-α-N-methylcarboxybetain, das durch die folgende Formel (2) dargestellt wird, und ein polymerisierbares Monomer, das durch die folgende Formel (3) dargestellt wird, umfasst, zur Durchführung einer Pfropfpolymerisation:

[chemische Formel 3]

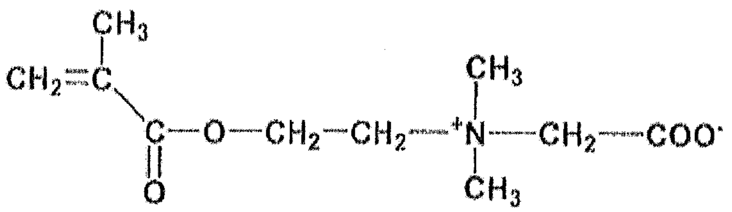

(2)

[chemische Formel 4]

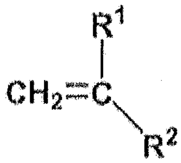

(3),

wobei in der Formel (3) $R^1$ für H oder $CH_3$ steht und $R^2$ für eine organische Gruppe, die eine elektrophile funktionelle Gruppe umfasst, steht.

9. Verfahren zur Herstellung eines Substrats als Ligandenträger gemäß Anspruch 8, wobei das polymerisierbare Monomer eine Epoxidgruppe aufweist.

10. Verfahren zur Herstellung eines Substrats als Ligandenträger gemäß Anspruch 8 oder 9, wobei es sich bei dem wasserunlöslichen Träger um eine poröse Membran oder um Teilchen handelt.

11. Adsorbens, bei dem ein Ligand, der aus der Gruppe ausgewählt ist, die aus Antikörpern, Proteinen, Peptiden und niedermolekularen Verbindungen besteht, an das Substrat als Ligandenträger gemäß einem der Ansprüche 1 bis 5 gebunden ist.

12. Blutbehandlungsgerät, bei dem das Innere eines Behälters, der mit einem Einlass zur Einleitung von Blut ins Innere und einem Auslass zum Ausleiten des Bluts nach außen ausgestattet ist, mit dem Adsorbens gemäß Anspruch 11 gefüllt ist.

## Revendications

1. Substrat pour support de ligand dans lequel un copolymère représenté par la formule (1) suivante est lié à au moins une surface d'un support insoluble dans l'eau :

[Formule chimique 1]

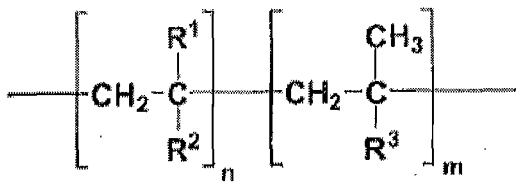

(1)

où dans la formule (1), n et m représentent un nombre entier positif, une valeur de m/(n + m) est supérieure ou égale à 0,1 et inférieure ou égale à 0,45, $R^1$ représente H ou $CH_3$, $R^2$ représente un groupe organique incluant un groupe fonctionnel électrophile, et $R^3$ représente

[Formule chimique 2]

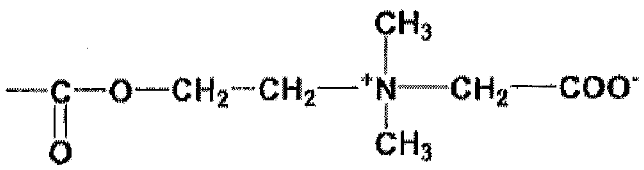

2. Substrat pour support de ligand selon la revendication 1, dans lequel un copolymère constitué de N-méthacryloyloxyéthyl-N,N-diméthylammonium-α-N-méthylcarboxybétaïne représentée par la formule (2) suivante et d'un monomère polymérisable représenté par la formule (3) suivante est lié à au moins une surface d'un support insoluble dans l'eau, dans lequel un rapport de composition molaire de l'unité de la N-méthacryloyloxyéthyl-N,N-diméthylammonium-α-N-méthylcarboxybétaïne au copolymère est supérieur ou égal à 0,1 et inférieur ou égal à 0,45 :

[Formule chimique 3]

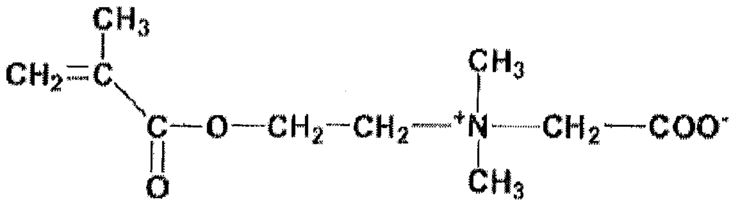

(2)

[Formule chimique 4]

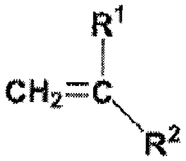

(3)

où dans la formule (3), $R^1$ représente H ou $CH_3$, et $R^2$ représente un groupe organique incluant un groupe fonctionnel électrophile.

3. Substrat pour support de ligand selon la revendication 1 ou 2, où le copolymère présente un groupe époxy.

4. Substrat pour support de ligand selon l'une quelconque des revendications 1 à 3, où la couverture de la surface de support insoluble dans l'eau avec le copolymère est supérieure ou égale à 27 %.

5. Substrat pour support de ligand selon l'une quelconque des revendications 1 à 4, où le support insoluble dans l'eau est une membrane ou des particules poreuses.

6. Utilisation d'un substrat comprenant un copolymère représenté par la formule (1) suivante lié à au moins une surface d'un support pour support de ligand insoluble dans l'eau :

[Formule chimique 1]

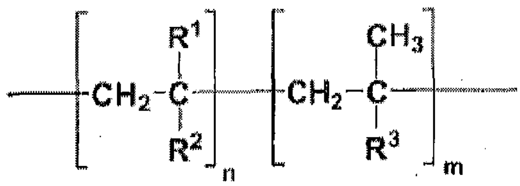

(1)

où dans la formule (1), n et m représentent un nombre entier positif, une valeur de m/(n + m) est supérieure ou égale à 0,1 et inférieure ou égale à 0,45, $R^1$ représente H ou $CH_3$, $R^2$ représente un groupe organique incluant un groupe fonctionnel électrophile, et $R^3$ représente

[Formule chimique 2]

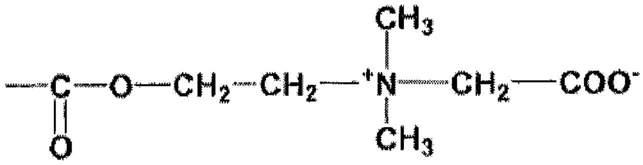

7. Utilisation d'un copolymère selon la revendication 6, où le copolymère présente un groupe époxy.

8. Procédé de production d'un substrat pour support de ligand selon la revendication 1, comprenant :

(i) une étape d'exposition d'un support insoluble dans l'eau à un rayonnement ; et
(ii) une étape d'immersion du support insoluble dans l'eau obtenu dans l'étape (i) dans une solution incluant de la N-méthacryloyloxyéthyl-N,N-diméthylammonium-$\alpha$-N-méthylcarboxybétaïne représentée par la formule (2) suivante et un monomère polymérisable représenté par la formule (3) suivante pour réaliser une polymérisation par greffage :

[Formule chimique 3]

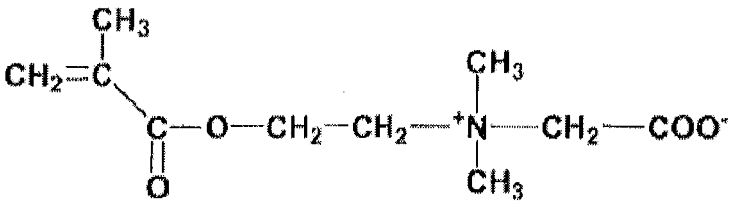

(2)

[Formule chimique 4]

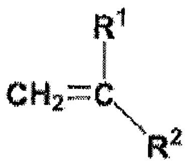

(3)

où dans la formule (3), $R^1$ représente H ou $CH_3$, et $R^2$ représente un groupe organique incluant un groupe fonctionnel électrophile.

9. Procédé de production d'un substrat pour support de ligand selon la revendication 8, où le monomère polymérisable présente un groupe époxy.

10. Procédé de production d'un substrat pour support de ligand selon la revendication 8 ou 9, où le support insoluble dans l'eau est une membrane ou des particules poreuses.

11. Adsorbant dans lequel un ligand choisi dans le groupe constitué d'anticorps, de protéines, de peptides et de composés de faible masse moléculaire est lié au substrat pour support de ligand selon l'une quelconque des revendications 1 à 5.

12. Equipement de traitement du sang dans lequel l'intérieur d'un récipient équipé d'une entrée pour introduire du sang à l'intérieur et d'une sortie pour décharger le sang vers l'extérieur est rempli avec l'adsorbant selon la revendication 11.

*Fig.1*

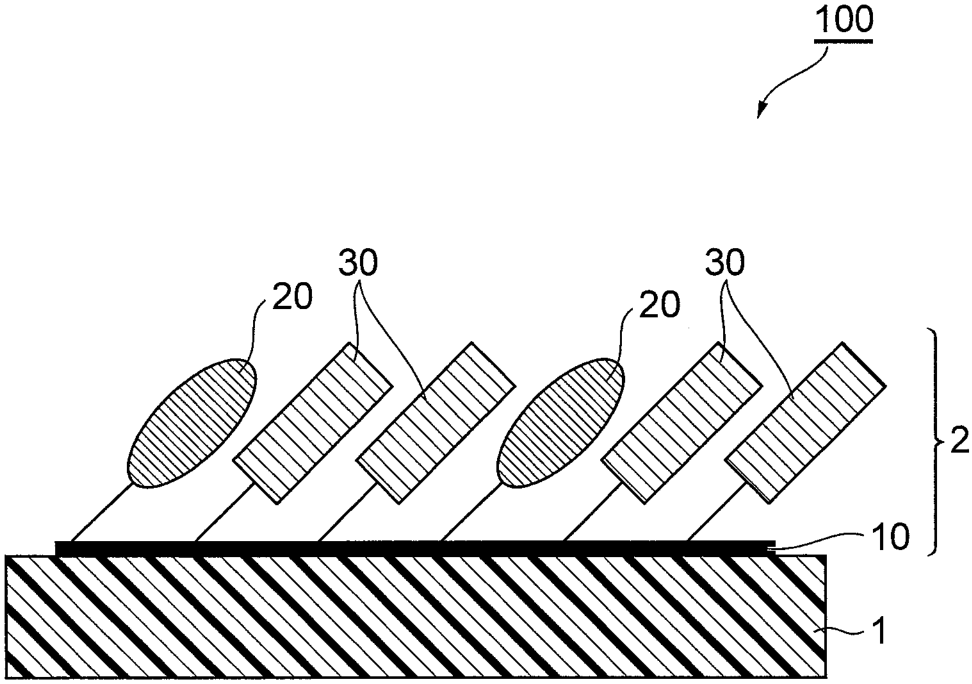

*Fig.2*

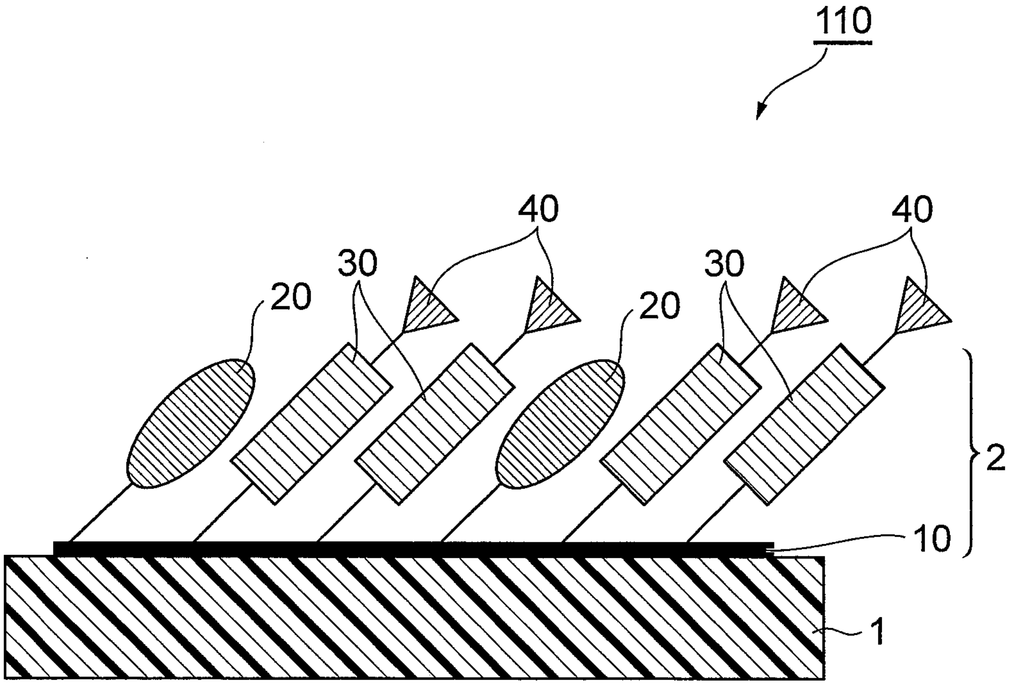

Fig.3

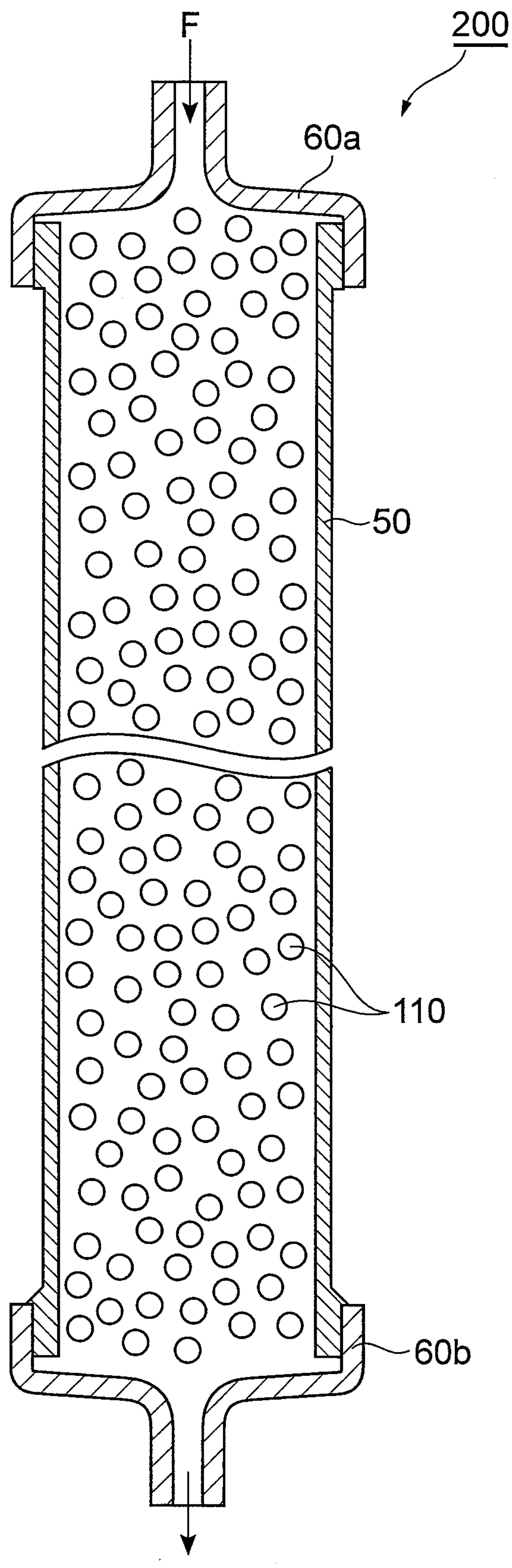

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 54063025 A
- JP 2000279512 A
- JP 9095474 A
- JP 9095586 A
- JP 11222470 A
- JP 2007130194 A
- JP H07300513 B
- WO 2005026224 A
- JP 1249063 A
- JP 3502094 A


### Non-patent literature cited in the description


- **A. HENGLEIN.** *Angew. Chem.,* 1955, vol. 70, 461
- **Y. OGIWARA.** *Poym. Sci., Polym Letter Ed.,* 1981, vol. 19, 457